# EUROPEAN PATENT SPECIFICATION

(11) **EP 3 595 662 B1**
(45) Date of publication and mention of the grant of the patent: **12.07.2023**
(21) Application number: 17728942.8
(22) Date of filing: 12.05.2017
(51) Int. Cl.: A61K 31/465, A61K 45/06, A61K 31/05, A61P 25/34

(54) **TOBACCO- AND SMOKE-LESS PRODUCTS CONSUMABLE BY HUMANS AS EPICUREAN OR MEDICAL PRODUCTS AND METHOD OF TREATING SMOKING ADDICTION**
TABAK- UND RAUCHFREIE PRODUKTE, DIE VOM MENSCHEN ALS GENUSSMITTEL- ODER MEDIZINISCHE PRODUKTE KONSUMIERT WERDEN, UND VERFAHREN ZUR BEHANDLUNG VON RAUCHSUCHT
PRODUITS SANS TABAC ET SANS FUMÉE CONSOMMABLES PAR DES ÊTRES HUMAINS EN TANT PRODUITS ÉPICURIENS OU MÉDICAUX ET PROCÉDÉ DE TRAITEMENT DE LA DÉPENDANCE AU TABAGISME

(30) Priority: 13.03.2017 NL 2018504
(43) Date of publication of application: 22.01.2020
(73) Proprietor: Pharma Unlimited B.V., 1012 KL Amsterdam (NL)
(72) Inventor: BAKKER, Keith Benjamin, 1012 KL Amsterdam (NL)
(74) Representative: Ferro, Frodo Nunes
(86) International application number: PCT/NL2017/050296
(87) International publication number: WO 2018/169387

(56) References cited:
- EP-A1- 2 233 134
- WO-A1-2017/189375
- WO-A1-2017/200740
- WO-A2-2006/060192
- WO-A2-2015/184127
- US-A1- 2015 057 341
- MORGAN CELIA J A ET AL: "Cannabidiol reduces cigarette consumption in tobacco smokers: Preliminary findings", ADDICTIVE BEHAVIORS, PERGAMON PRESS, OXFORD, GB, vol. 38, no. 9, 1 April 2013 (2013-04-01), pages 2433-2436, XP028564804, ISSN: 0306-4603, DOI: 10.1016/J.ADDBEH.2013.03.011 cited in the application
- DATABASE BIOSIS [Online] BIOSCIENCES INFORMATION SERVICE, PHILADELPHIA, PA, US; September 2002 (2002-09), COHEN C ET AL: "SR141716, a central cannabinoid (CB1) receptor antagonist, blocks the motivational and dopamine-releasing effects of nicotine in rats.", XP002775264, Database accession no. PREV200300004475 & BEHAVIOURAL PHARMACOLOGY, vol. 13, no. 5-6, September 2002 (2002-09) , pages 451-463, ISSN: 0955-8810
- BERNARD LE FOLL ET AL: "Blocking cannabinoid CB1 receptors for the treatment of nicotine dependence: insights from pre-clinical and clinical studies : CB1 antagonists for nicotine addiction", ADDICTION BIOLOGY, vol. 13, no. 2, 1 June 2008 (2008-06-01), pages 239-252, XP055420969, GB ISSN: 1355-6215, DOI: 10.1111/j.1369-1600.2008.00113.x

## Description

### Field of the invention

The invention relates to tobacco- and smokeless products consumable by humans which release nicotinic agonists into a human body in a dose tolerable thereby and which produce effects similar to nicotine released into the body by smoking tobacco.

### Background of the invention

A wide variety of tobacco- and smokeless products is known which upon human consumption release nicotinic agonists into a human body in a dose tolerable thereby and which produce effects similar to nicotine released into the body by smoking tobacco.

The products can be epicurean, non-medical, products used as an indulgence to provide the human body with sensations similar to that of smoking tobacco, and as an alternative thereto without any treatment of smoking or nicotine addiction. Examples of such alternatives to smoking tobacco but with less health-related negative side-effects are e.g. e-cigarettes or vaporizers with vaporizing liquids containing nicotine in a suitable form. These release nicotine, without burning, by vaporizing the liquid containing nicotine. The vapour with the nicotine is subsequently inhaled and absorbed in the human body.

However, a disadvantage of the known products is that the human body does not experience a sensation equivalent to that of the nicotine administered to the body by smoking tobacco. This in particular when the product is used by a human accustomed to smoking tobacco.

BERNARD LE FOLL ET AL, "Blocking cannabinoid CB1 receptors for the treatment of nicotine dependence: insights from pre-clinical and clinical studies : CB1 antagonists for nicotine addiction", ADDICTION BIOLOGY, GB, (20080601), vol. 13, no. 2 discloses that in experimental animals, cannabinoid CB₁ receptor antagonists Rimonabant (SR141716) and AM251 block nicotine self-administration behaviour, and that this may be related to the blockade of the dopamine-releasing effects of nicotine in the brain. This prior art document further discloses that it seems that Rimonabant is an efficacious treatment for smoking cessation, although its efficacy doesn't exceed that of nicotine replacement therapy and its use may be limited by emotional side effects (nausea, anxiety and depression, mostly). Le Foil et all. further discloses that the efficacy of rimonabant in a situation where subjects have been exposed chronically to nicotine is questioned.

COHEN C ET AL, "SR141716, a central cannabinoid (CB1) receptor antagonist, blocks the motivational and dopamine-releasing effects of nicotine in rats.", BIOSIS, BIOSCIENCES INFORMATION SERVICE, PHILADELPHIA, PA, US, (200209) discloses a study on the effects of the selective CB(1) receptor antagonist SR141716 on the motivational effects of nicotine in the rat. Administration of SR141716 (0.3 and 1 mg/kg) was found to decrease nicotine self-administration (0.03 mg/kg/injection). Secondly, using brain microdialysis, SR141716 (1-3 mg/kg) was found to block nicotine-induced dopamine release in the shell of the nucleus accumbens (NAc) and the bed nucleus of the stria terminalis.

Morgan et all. (MORGAN CELIA J A ET AL, "Cannabidiol reduces cigarette consumption in tobacco smokers: Preliminary findings", ADDICTIVE BEHAVIORS, PERGAMON PRESS, OXFORD, GB, (20130401), vol. 38, no. 9, doi:10.1016/J.ADDBEH.2013.03.011, ISSN 0306-4603, pages 2433 - 2430 discloses a pilot, randomised double blind placebo controlled study set out to assess the impact of the ad-hoc use of cannabidiol (CBD) in smokers who wished to stop smoking. 24 smokers were randomised to receive an inhaler of CBD (n=12) or placebo (n=12) for one week, they were instructed to use the inhaler when they felt the urge to smoke. Those treated with CBD reduced the number of cigarettes smoked by ~40% during treatment. Morgan et all. discloses that this reduction was not maintained at follow-up after the one-week treatment.

In addition, tobacco- and smokeless medical products are known which are used as part of treating nicotine addiction induced by smoking tobacco.

United States patent application publication US2015057341 discloses a composition deliverable to a user via vaporization that includes a cannabinoid capable of inducing a pharmacological effect, an ester, a condensation aerosol, and a carrier liquid solution of food grade materials. The cannabinoid may include a bio-active ingredient receivable by a cannabinoid receptor and/or an acetylcholine receptor. The pharmacological effect may be produced by reception of tetrahydrocannabinol, cannabidiol, or mixtures thereof. US2015057341 discloses that a vaporized form of the composition may assist with smoking cessation, and that to ease the transition away from tobacco products, the product may contain a tobacco essence, nicotine free tobacco essence, and/or flavouring.

European patent application publication EP2233134 discloses an intra-oral dosage form, as well as methods and systems for delivering nicotine and/or metabolites thereof, such as cotinine, nicotine N'-oxide, nornicotine, (S)-nicotine-N-β-glucuronide and mixtures, isomers, salts and complexes thereof and/or a nicotine-mimicking compound so as to obtain a transmucosal uptake of nicotine and/or metabolites thereof and mixtures, isomers, salts and complexes thereof in the oral cavity of the subject, as well as a method for producing the intra-oral dosage form.

The multi portion intra-oral dosage form has a discernible difference in organoleptic sensation between at least two portions, such as flavour, effervescence, olfactory sensation, viscosity, elasticity, rheology, texture, mouth feel and difference in disintegration rate. Nicotine in any form and/or a nicotine-mimicking compound may be included in one or several portions of the dosage form. EP2233134 discloses that the association of a certain organoleptic sensation with a certain functionality provides means for increased awareness of the functionality and constituents of the dosage form and hence increase the patient's compliance to the medication as well as provide means for improving current therapy and efficacy.

For example, medical products, such as transdermal patches or chewing gum, are known which are used in nicotine replacement therapy, i.e. to administer to the human body nicotine in a gradually lowering dose to treat nicotine addiction. For example, Fiore M.C., Smith S.S., Jorenby D.E., Baker T.B.: "The Effectiveness of the Nicotine Patch for Smoking Cessation A Meta-analysis", JAMA. 1994;271(24) :1940-1947, describes a nicotine patch. This publication reports that across 17 studies (n=5098 patients) meeting inclusion criteria, overall abstinence rates for the active patch were 27% at the end of treatment and 22% at 6 months. Said differently, the overwhelming majority of patients does not successfully stop smoking.

Thus, the treatments in which these known medical products are used have an unsatisfactory rate of people succeeding to stop smoking.

### Summary of the invention

The present invention provides products as described in the accompanying claims.

Specific embodiments of the invention are set forth in the dependent claims.

These and other aspects of the invention will be apparent from and elucidated with reference to the embodiments described hereinafter.

### Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

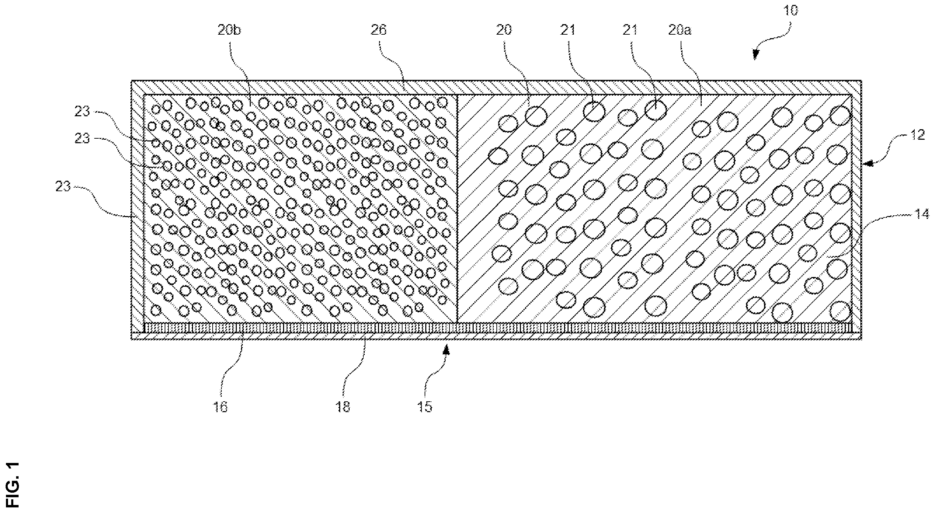

FIG. 1 schematically shows a cross-sectional view of a transdermal patch.

### Detailed description of the preferred embodiments

In the following, details will not be explained in any greater extent than that considered necessary for the understanding and appreciation of the underlying concepts of the present invention and in order not to obfuscate or distract from the teachings of the present invention.

### The product

The examples hereinafter relate to tobacco- and smoke-less products, e.g. in a form suitable to administer additives non-invasively to the human body, using topical (skin) routes.

The product comprises a first quantity of a first compound in a form suitable to release in-vivo in a human body a dose of a nicotinic agonist tolerable by the human body and sufficient to provide a sensation similar to that of nicotine released into the body by smoking tobacco. The product further comprises a second quantity of a second compound in a form suitable to release in-vivo in the human body a dose of a non-psychoactive cannabinoid tolerable by the human body which reduces the desire to smoke tobacco.

If the product is used to treat tobacco smoking addiction, this composition is likely to yield a higher success rate because it at least partially lowers the barrier to successfully quit smoking, which is believed to be caused by continuing mental discomfort. In particular, the nicotinic agonist allows to reduce the mental discomfort related to a lack of nicotine in the human body while the non-psychoactive cannabinoid allows to inhibit, or at least reduce, the mental discomfort caused by the lack of the habits and emotions the tobacco smoker is addicted to, e.g. cue induced or otherwise related to a craving for smoking tobacco.

The first compound and the second compound can be provided on a common carrier, such a e.g. embedded in a matrix compound or in separate dosing units, as patches, which allow to separately administer the nicotinic agonist and the non-psychoactive cannabinoid to the human body.

The product can be provided in a kit of several products, as transdermal patches, with a similar dose of nicotinic agonist and the cannabinoid per product, or with differing doses. The kit can comprise a common package for the products such as a paper- or cardboard box.

For example, the kit can contain at least two products with the quantity of the first compound and/or form of the first compound differing between the products to release different doses of the nicotinic agonist. If the quantities of the first and second compound are on a common carrier, the products may contain the second compound in a quantity and/or form which is the same to release the same dose per product or which differs between the products to release doses of cannabinoid which differ per product. The kit can comprise in addition products with only a quantity of the second compound and not the first compound or vice versa.

### Non-psychoactive cannabinoid

In a non-claimed aspect of the disclosure, the non-psychoactive cannabinoid to be released can be any non-psychoactive cannabinoid suitable to reduce the desire to smoke tobacco. The non-psychoactive cannabinoid can be selected out the cannabinoids with a metabolic pathway in the human body which differs (at least partially) from a metabolic pathway of tetrahydrocannabinol, to ensure a good inhibition of psychoactive effects. The non-psychoactive cannabinoid can be of a type which acts on a metabolic pathway of endogenous (to the human body) cannabinoid ligands to effectively suppress the need to smoke tobacco, and can e.g. be an antagonist to CB1 and/or CB2 receptors in the human body, such as an indirect antagonist of exogenous (to the human body) CB1 and/or CB2 agonists. Without wishing to be bound to theory, it is believed that a non-psychoactive cannabinoid which increases a concentration in the human body of cannabinoid ligands of CB1 and/or CB2 receptors endogenous to the human body effectively diminishes a craving for nicotine and/or other addictive substances administered to the human body by smoking tobacco.

The non-psychoactive cannabinoid can be a phytocannabinoid. Phytocannabinoids are the cannabinoids derived from cannabis plants. Cannabinoids are a group of chemicals known to activate cannabinoid receptors in cells, of which endocannabinoids are endogenous cannabinoids found in humans and other animals and exocannabinoids are exogenous to the human body.

The phytocannabinoids can be isolated from cannabis plants or produced synthetically. When isolating the phytocannabinoids from plants they can be purified to the extent that except for trace quantities thereof all of the other naturally occurring compounds, such as other minor cannabinoids and plant molecules, such as terpenes, are removed. This purification results in a purity of greater than 99% (w/w) of the target cannabinoid.

The phytocannabinoid can be in the form of a botanical drug substance (BDS). A "botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug derived from one or more plants, algae, or microscopic fungi. It is prepared from botanical raw materials by one or more of the following processes: pulverisation, decoction, expression, aqueous extraction, ethanolic extraction or other similar processes*."*

A botanical drug substance does not include a highly purified or chemically modified substance derived from natural sources. Thus, BDS derived from cannabis plants do not include highly purified pharmacopoeial grade cannabinoids and will containe a principle phytocannabinoid and other phytocannabinoids.

The "principle phytocannabinoid" in a BDS is the phytocannabinoid that is present in an amount that is higher than that of the other phytocannabinoids. The product can comprise BDS with a non-psychoactive cannabinoid as the principle phytocannabinoid present in an amount greater than 40% (w/w) of the total extract. More preferably the principle phytocannabinoid is present in an amount greater than 50% (w/w) of the total extract. More preferably still the principle phytocannabinoid is present in an amount greater than 60% (w/w) of the total extract.

The amount of the principle phytocannabinoid in the BDS is preferably greater than 75% of the phytocannabinoid-containing fraction, more preferably still greater than 85% of the phytocannabinoid-containing fraction, and more preferably still greater than 95% of the phytocannabinoid-containing fraction.

Preferably, the BDS has a THC content below the Lowest Observed Effect threshold, such as less than 0.3% w/w, more preferably less than 0.2% w/w (measured in accordance with annex C to European Directive 1164/89). This is generally considered to be non-psychotropic and allows a product which is safe as a product. Generally speaking it is advantageous is if the content of psycho-active cannabinoids in the product is below the regulatory threshold, and for practical purposes negligible.

According to the present invention, the non-psychoactive cannabinoid is cannabidiol, also referred to as CBD (2-[(1R,6R)-3-methyl-6-(prop-1-en-2-yl)cyclohex-2-en-1-yl]-5-pentylbenzene-1,3-diol), which has the following structural formula:

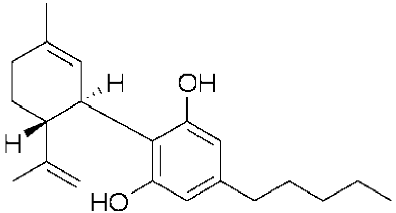

Mechoulam, L. Hanus, "Cannabidiol: an overview of some chemical and pharmacological aspects. art I: chemical aspects", Chemistry and Physics of Lipids, 121 (2002) 35-43 describes the synthesis of CBD. The product may e.g. comprise a naturally occurring CBD isomer, such as A¹-cannabidiol.

In Morgan et all, "Cannabidiol reduces cigarette consumption in tobacco smokers: Preliminary findings", Addictive Behaviors, Volume 38, Issue 9, Pages 2433-2436, a treatment with CBD (as aerosol with an inhaler administered a dose of 400 µg CBD dissolved in absolute ethanol ≈5%) has been found to be particularly effective in reducing the number of cigarettes smoked with 40%.

In a non-claimed aspect of the disclosure, the cannabinoid may also be a synthetic derivative of CBD, such as CBD-DMH (CBD di-methylheptyl), although other cannabinoids can be used as well.

### Nicotinic agonist

In a non-claimed aspect of the disclosure, the nicotinic agonist to be released can be any type of nicotinic agonist suitable to substitute for nicotine released into the body by smoking tobacco. The agonist can be a full agonist or a partial agonist. According to the present invention, the nicotinic agonist is nicotine, 3-[(2S)-1-methylpyrrolidine-2-yl]pyridine, with the following chemical structure:

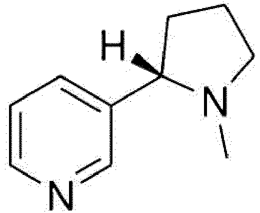

For example, the nicotinic agonist may be nicotine in its naturally occurring form: (-)-nicotine.

### The medical product

In the medical product, the substances used can be of a pharmacopeal grade. In this example, the product comprises the first compound in a pharmaceutically acceptable form suitable to release a dose of nicotinic agonist tolerable by the human body and effective to treat symptoms of a nicotine deficiency in the body induced by the human body being deprived from smoking tobacco, and the second compound is in a pharmaceutically acceptable form suitable to release in-vivo in the human body a dose of non-psychoactive cannabinoid tolerable by the human body and effective to treat a craving for smoking tobacco.

In a first example, the product is a transdermal patch which can be is placed on the skin to deliver a specific dose of medication through the skin and into the bloodstream. The transdermal patch allows a controlled release of the nicotinic agonist and the cannabinoid into the human body. The patch may e.g. comprise a porous membrane covering a first reservoir with the nicotinic agonist, or precursor, thereof in a pharmaceutically acceptable form and a second reservoir with the cannabinoid, or a precursor thereof, in a pharmaceutically acceptable form.

Referring to FIG. 1, there is shown a cross-sectional view of the first example, which is a transdermal patch 10. The patch 10 comprises a wall, or backing layer, 12 with a top face portion 26. The wall 12 provides a reservoir 14 having an open bottom end 15 and an opposing top end 17. The wall 12 can be composed of a medically approved plastic material including plastic composites formed by any suitable technique. Other suitable materials, generally of plastic polymeric composition, can be used for the wall 12 and are known.

The reservoir 14 includes a matrix 20 capable of suspending a drug for subsequent release. Suitable formulations are e.g. guar, acacia, or xanathan gum, or a gelling agent or polymer such as carboxypolymethylene, hydroxyethylcellulose or polyacrylamide. The matrix 20 includes a first region 20 a and a second region 20 b located at the top end 17 of the reservoir 14. The first and second regions 20 a and 20 b can be composed of the same material or different materials exhibiting different diffusion rates and/or chemical properties. First region 20a thus forms a first reservoir for the first compound, and second region 20b thus forms a second reservoir for the second compound. The sensitivity of the matrix material to the permeation of moisture is controlled by the choice of materials or formulation. The matrix 20 is designed to allow moisture, ions, electrolytes and the like, typically, present in perspiration, to diffuse or permeate in order to release the substances for delivery to, and subsequent passage through the skin.

In the example, the first region 20a of the matrix 20 comprises the nicotinic agonist 21, or a precursor thereof, suspended therein. The second region 20b of the matrix 20 comprises the non-psychoactive cannabinoid, or a precursor thereof, suspended therein. In both regions, the substances are in a pharmaceutically acceptable form. The first and second matrix regions 20a and 20b can be suitably formulated and positioned with one another to provide rates and times of delivery as desired.

The matrix 20 is maintained in contact with the patient's skin during administration via a permeable adhesive layer 16. The permeable adhesive layer 16 can be composed of a suitable pressure-sensitive adhesive material, and is located at the bottom end 15 of the reservoir 14 overlaying the bottom portion of the matrix 20. The adhesive layer 16 enables the matrix 20 and the wall 12 to be secured to the skin of the patient, while permitting free passage of molecules (e.g. perspiration and drugs) in between the patch 10 and the patient's skin. It will be understood that when the patch 10 is provided to the patient, the adhesive layer 16 is normally covered with a disposable protective layer 18 that the patient must remove prior to application. When attached to the skin of the patient, the wall 12 provides an occlusive covering, which enhances hydration of the skin area covered by the patch 10, and diffusion of the perspiration into the matrix 20. Hydration of the skin fosters release and absorption of the first and second compounds. Alternatively, substantial portions of the adhesive layer 16 can be removed or eliminated to provide direct contact of the matrix 20 with the patient's skin for enhancing ease of delivery of the substances into the human body.

The material of the matrix regions 20 a and 20 b can be selected or formulated to control of the rate of drug release from the matrix 20, as well as the diffusion rate of the perspiration through which the corresponding matrix regions 20 a and 20 b are activated for release of their associated pharmaceutical agent such as agonist 21 and cannabinoid 23, respectively. The matrix regions 20 a or 20 b can be formulated to be relatively impervious to moisture, for example, one that is thicker or less permeable because of its physico-chemical properties, or one that contains a higher content of hydrophobic elements in its composition, will result in a more gradual drug release over a sustained time period and gradual diffusion of the patient's perspiration there through. In contrast, a matrix region 20 a or 20 b that is relatively permeable to water will rapidly release the drug over a relatively shorter time period.

Once the patch 10 is properly applied to the patient's skin, the occlusive wall 12 entraps the patient's perspiration produced from the covered area of the skin. The perspiration permeates through the adhesive layer 16 into the first matrix region 20 a and the second matrix 20b. As the perspiration solvates the matrix material, the suspended agonist 21 is released and flows to the patient's skin for delivery. As the perspiration flows into the second matrix region 20 b, the cannabinoid 23 is released therefrom and begins to diffuse through the matrix 20 toward the patient's skin.

The transdermal patch 10, as described previously, includes the matrix 20 with two regions 20 a and 20 b, each exhibiting individual diffusion characteristics. The matrix 20 urges the nicotinic agonist 21 and the cannabinoid 23 to controlled flow towards the patient's skin based on the concentration gradient. Similarly, the perspiration including moisture, ions, electrolytes and other secretions, produced by the patient, flow into the matrix 20.

Thus, when applied to the skin, the patch delivers a steady dose of nicotinic agonist and cannabinoid over a prolonged period of time, e.g. 12, 24 or 48-hours. By using a series of patches over a period of time, such as several weeks, with the dose of nicotinic agonists per patch lowering in time, e.g. per patch or for a sequence of patches, the nicotine dose can be reduced gradually until the patient no longer craves for nicotine and/or smoking tobacco, and they can stop using the medication. The patient can choose to continue using a maintenance dose of CBD as needed if they choose to do so using other delivery methods. In this respect, the dose of CBD per patch may remain constant over time or vary over time.

The quantities of the first compound and the second compound in the products may be such that they can be used in the following dosage forms & strengths may be as follows:
7 mg nicotine and 10 mg CBD per 24 hours;
14 mg nicotine and 10 mg CBD per 24 hours;
21 mg day nicotine and 10 mg CBD per 24 hours.

For instance, in case the product are individual transdermal patches, each patch may contain the amount to be released in 24 hours. However, it will be apparent that the units may e.g. be administered in a different rhythm, for example 1 per 12 hours or otherwise and that therefore the amount of substance be adjusted proportionally.

### Smoking Cessation program

The medical products described can be used to treat tobacco smoking addiction. Such a treatment can comprise administering the nicotinic agonist, in a form suitable to release in-vivo in a human body a dose tolerable by the human body and effective as a treatment to symptoms of the nicotine deficiency in the body induced by the human body being deprived from smoking tobacco; and the non-psychoactive cannabinoid, in a form suitable to release in-vivo in the human body a dose tolerable by the human body and effective as a treatment of a craving to smoke tobacco.

It is proposed that the initial dosage is, inter alia, based on how heavily the patient smoked, e.g. in number of cigarettes or equivalent measures. This will provide an initial adequate replacement for the nicotine consumed by smoking tobacco. The initial dose and duration of therapy can depend on a number of factors such as weight, number of cigarettes smoked, and various medical conditions. For example, the dosage can range from 7 mg to 21 mg of nicotinic agonist administered to the patient's body.

In this example, the dosage is lowered over time to allow the body to gradually adjust to less nicotine and can step down to a lower strength until the patient is gradually able to taper off the nicotine replacement patches altogether. As the cannabinoid, in particular CBD, is not addictive but highly effective for stress reduction, a common cause for relapse, the use thereof can be continued even after the nicotine is out of the system.

Therapy for most people will begin with one 21 mg nicotine and 10 mg CBD patch per day for 6 weeks. The next stage of therapy will gradually reduce the dose of nicotine but the CBD dose will remain at 10 mg.

After successful completion of the first 6-week stage, the 14 mg nicotine and 10mg CBD patch is started for 2 weeks, immediately followed by the 7 mg/day patch for another 2 weeks. Treatment will generally take 8 to 12 weeks. The medication may e.g. be taken for 3 months or less. The patient has to stop smoking completely when taking this medication.

Although other dosing regimens may be found to be suitable, it is proposed that the following treatment be used, in which the dosing depends on the number of cigarettes (or cigarette equivalents) smoked per day before the treatment as follows:

### 20 or more cigarettes/day - 21/14/7-mg regimen:

21-mg nicotine+ 10 mg CBD per 24 hours for 6 weeks,
14-mg nicotine + 10mg CBD per 24 hours for 2 weeks,
7-mg nicotine +10 mg CBD per 24 hours for 2 weeks.
10 or more cigarettes/day- 14/7-mg regimen:
14-mg nicotine+ 10 mg CBD per 24 hours for 6 weeks,
7-mg nicotine + 10 mg CBD per 24 hours for 2 weeks.

The dose is provided through a transdermal patch to ensure a gradual release over the 24 hours.

In the foregoing specification, the invention has been described with reference to specific examples of embodiments of the invention. It will, however, be evident that various modifications and changes may be made therein without departing from the broader scope of the invention as set forth in the appended claims.

For instance, although in the examples nicotine is used as a suitable nicotinic agonist, pharmaceutically acceptable salts, resins and complexes, of nicotine and/or cannabidiol may be used as a precursor thereof. For example, nicotine salts such as nicotine bitartrate, nicotine hydrochloride, nicotine dihydrochloride or nicotine sulfate, or other nicotine actives may be used.

Furthermore, although in some examples the product only contains a single cannabinoid it will be apparent that the product may comprise several cannabinoids without the product itself exhibiting noticeable psychoactivity, e.g. by containing trace quantities of psychoactive cannabinoids, such as when the cannabinoid is a botanical drug substance with non-psychoactive cannabinoids as principle phytocannabinoid.

In the claims, any reference signs placed between parentheses shall not be construed as limiting the claim. The word 'comprising' does not exclude the presence of other elements or steps then those listed in a claim. Furthermore, the terms "a" or "an," as used herein, are defined as one or more than one.

Also, the use of introductory phrases such as "at least one" and "one or more" in the claims should not be construed to imply that the introduction of another claim element by the indefinite articles "a" or "an" limits any particular claim containing such introduced claim element to inventions containing only one such element, even when the same claim includes the introductory phrases "one or more" or "at least one" and indefinite articles such as "a" or "an." The same holds true for the use of definite articles. Unless stated otherwise, terms such as "first" and "second" are used to arbitrarily distinguish between the elements such terms describe. Thus, these terms are not necessarily intended to indicate temporal or other prioritization of such elements The mere fact that certain measures are recited in mutually different claims does not indicate that a combination of these measures cannot be used to advantage.

## Claims

1. A tobacco- and smokeless product for use in treatment of tobacco smoking addiction, comprising:
a first quantity of nicotine or a pharmaceutically acceptable salt, resin or complex thereof, in a form suitable to release in-vivo in a human body a dose of nicotine, which dose is tolerable by the human body and effective to treat symptoms of a nicotine deficiency induced by the human body being deprived from smoking tobacco; and
a second quantity of cannabidiol or a pharmaceutically acceptable salt, resin or complex thereof, in a form suitable to release in-vivo in the human body a dose of cannabidiol, which dose is tolerable by the human body and effective to treat a craving for smoking tobacco;
said treatment comprising:
administering via a transdermal patch the dose of nicotine to treat the symptoms of the nicotine deficiency and the dose of cannabidiol to treat the craving for smoking tobacco.

2. Product for use according to claim 1, wherein the treatment comprises using the transdermal patch placed on the skin to deliver a specific dose of medication through the skin and into the bloodstream.

3. Product for use according to claim 1 or 2, wherein the treatment comprises the patient having to stop smoking completely when taking the medication.

4. Product for use according to one or more of the preceding claims, wherein the treatment comprises continuing use of cannabidiol, or the pharmaceutically acceptable salt, resin or complex thereof, after the nicotine is out of the system.

5. Product for use according to any one of the preceding claims, wherein the cannabidiol, or the pharmaceutically acceptable salt, resin or complex thereof, is produced from cannabis plants.

6. Product for use according to any one of the claims 1-4, wherein the cannabidiol, or the pharmaceutically acceptable salt, resin or complex thereof, is a synthetic cannabinoid.

7. Product for use according to any one of the preceding claims, wherein the treatment comprises: releasing the nicotine in a dose which lowers over time to get the human body gradually accustomed to the absence of extraneous nicotine administered by smoking tobacco.

8. Product for use according to claim 7, wherein the treatment comprises:
releasing the cannabidiol in a dose which remains the same for consecutive products, and preferably constant during the time the nicotine is released.

9. Product for use according to claim 7 or 8, wherein the treatment comprises:
using a series of patches over a period of time, with the dose of nicotine per patch lowering in time.

10. Product for use according to claim 9, wherein the nicotine dose is reduced gradually until the patient no longer craves for nicotine and smoking tobacco and stops using the medication.

11. Product for use according to claim 9 or 10, wherein the treatment comprises the patient continuing using a maintenance dose of cannabidiol.

12. Product for use according to any one of the preceding claims, comprising several cannabinoids without the product itself exhibiting noticeable psychoactivity.

13. Kit of at least two products for use according to any one of the preceding claims, wherein the quantity of nicotine or the pharmaceutically acceptable salt, resin or complex thereof and/or form of nicotine or the pharmaceutically acceptable salt, resin or complex thereof differs between the products to release different doses of nicotine.

14. Kit for use according to claim 13, comprising a common package in which the products are provided.

## Patentansprüche

1. Tabak- und rauchloses Produkt zur Verwendung bei der Behandlung von Tabakrauchsucht, umfassend:
eine erste Menge an Nicotin oder eines pharmazeutisch unbedenklichen Salzes, Harzes oder Komplexes davon in einer zur in-vivo-Freisetzung einer Nicotindosis in einem menschlichen Körper geeigneten Form, wobei die Dosis für den menschlichen Körper verträglich und zur Behandlung von Symptomen eines dadurch, dass dem menschlichen Körper das Rauchen von Tabak entzogen wird, induzierten Nicotinmangels wirksam ist, und
eine zweite Menge an Cannabidiol oder eines pharmazeutisch unbedenklichen Salzes, Harzes oder Komplexes davon in einer zur in-vivo-Freisetzung einer Cannabidioldosis im menschlichen Körper geeigneten Form, wobei die Dosis für den menschlichen Körper verträglich und zur Behandlung des Verlangens, Tabak zu rauchen, wirksam ist,
wobei die Behandlung Folgendes umfasst:
die Verabreichung der zur Behandlung der Symptome des Nikotinmangels erforderlichen Nikotindosis und der zur Behandlung des Verlangens, Tabak zu rauchen, erforderlichen Cannabidioldosis mittels eines transdermalen Pflasters.

2. Produkt zur Verwendung nach Anspruch 1, wobei die Behandlung die Anwendung eines auf der Haut angebrachten transdermalen Pflasters zur Verabreichung einer spezifischen Dosis an Medikament über die Haut und in den Blutstrom umfasst.

3. Produkt zur Verwendung nach Anspruch 1 oder 2, wobei der Patient bei der Behandlung während der Einnahme des Medikaments vollständig mit dem Rauchen aufhört.

4. Produkt zur Verwendung nach einem oder mehreren der vorhergehenden Ansprüche, wobei die Behandlung die fortgesetzte Anwendung von Cannabidiol bzw. dem pharmazeutisch unbedenklichen Salz, Harz oder Komplex davon nach dem Eliminieren des Nicotins aus dem Körper umfasst.

5. Produkt zur Verwendung nach einem der vorhergehenden Ansprüche, wobei das Cannabidiol bzw. das pharmazeutisch unbedenkliche Salz, das pharmazeutisch unbedenkliche Harz oder der pharmazeutisch unbedenkliche Komplex davon von Cannabispflanzen produziert wird.

6. Produkt zur Verwendung nach einem der Ansprüche 1-4, wobei es sich bei dem Cannabidiol bzw. dem pharmazeutisch unbedenklichen Salz, Harz oder Komplex davon um ein synthetisches Cannabinoid handelt.

7. Produkt zur Verwendung nach einem der vorhergehenden Ansprüche, wobei die Behandlung Folgendes umfasst:
die Freisetzung des Nicotins in einer im Verlauf der Zeit geringer werdenden Dosis, um den menschlichen Körper schrittweise an das Fehlen des durch Rauchen von Tabak von außen zugeführten Nicotins zu gewöhnen.

8. Produkt zur Verwendung nach Anspruch 7, wobei die Behandlung Folgendes umfasst:
die Freisetzung des Cannabidiols in einer Dosis, die für aufeinanderfolgende Produkte gleich bleibt und vorzugsweise während der Zeit, in der das Nicotin freigesetzt wird, konstant bleibt.

9. Produkt zur Verwendung nach Anspruch 7 oder 8, wobei die Behandlung Folgendes umfasst:
die Anwendung einer Reihe von Pflastern über eine Zeitspanne, wobei die Dosis an Nicotin pro Pflaster im Verlauf der Zeit abnimmt.

10. Produkt zur Verwendung nach Anspruch 9, wobei die Nicotindosis schrittweise gesenkt wird, bis der Patient kein Verlangen mehr für Nicotin und das Rauchen von Tabak hat und die Anwendung des Medikaments absetzt.

11. Produkt zur Verwendung nach Anspruch 9 oder 10, wobei die Behandlung die fortgesetzte Anwendung einer Erhaltungsdosis an Cannabidiol durch den Patienten umfasst.

12. Produkt zur Verwendung nach einem der vorhergehenden Ansprüche, umfassend mehrere Cannabinoide, ohne dass das Produkt selbst eine erkennbare Psychoaktivität zeigt.

13. Kit mit mindestens zwei Produkten zur Verwendung nach einem der vorhergehenden Ansprüche, wobei die Menge an Nicotin bzw. dem pharmazeutisch unbedenklichen Salz, Harz oder Komplex davon und/oder die Form des Nicotins bzw. des pharmazeutisch unbedenklichen Salzes, Harzes oder Komplexes davon sich zwischen den Produkten unterscheidet, so das unterschiedliche Dosen an Nicotin freigesetzt werden.

14. Kit zur Verwendung nach Anspruch 13, umfassend eine gemeinsame Verpackung, in der die Produkte bereitgestellt werden.

## Revendications

1. Produit sans tabac et sans fumée pour une utilisation dans le traitement d'une addiction au tabagisme, comprenant :
une première quantité de nicotine ou d'un sel, d'une résine ou d'un complexe pharmaceutiquement acceptable correspondant(e), sous une forme appropriée pour libérer *in vivo* dans un corps humain une dose de nicotine, laquelle dose étant tolérable par le corps humain et efficace pour traiter des symptômes d'un manque de nicotine induits par le corps humain privé du fait de fumer du tabac ; et
une deuxième quantité de cannabidiol ou d'un sel, d'une résine ou d'un complexe pharmaceutiquement acceptable correspondant(e), sous une forme appropriée pour libérer *in vivo* dans le corps humain une dose de cannabidiol, laquelle dose étant tolérable par le corps humain et efficace pour traiter un état de manque pour le fait de fumer du tabac ;
ledit traitement comprenant :
une administration via un patch transdermique de la dose de nicotine pour traiter les symptômes du manque de nicotine et de la dose de cannabidiol pour traiter l'état de manque pour le fait de fumer du tabac.

2. Produit pour une utilisation selon la revendication 1, le traitement comprenant une utilisation du patch transdermique placé sur la peau pour apporter une dose spécifique de médicament à travers la peau et dans le flux sanguin.

3. Produit pour une utilisation selon la revendication 1 ou 2, le traitement comprenant le fait que le patient doit arrêter de fumer complètement lors de la prise du médicament.

4. Produit pour une utilisation selon l'une ou plusieurs des revendications précédentes, le traitement comprenant la poursuite de l'utilisation de cannabidiol, ou du sel, de la résine ou du complexe pharmaceutiquement acceptable correspondant(e), après que la nicotine est en dehors du système.

5. Produit pour une utilisation selon l'une quelconque des revendications précédentes, le cannabidiol, ou le sel, la résine ou le complexe pharmaceutiquement acceptable correspondant(e), étant produit(e) à partir de végétaux de cannabis.

6. Produit pour une utilisation selon l'une quelconque des revendications 1 à 4, le cannabidiol, ou le sel, la résine ou le complexe pharmaceutiquement acceptable correspondant(e), étant un cannabinoïde synthétique.

7. Produit pour une utilisation selon l'une quelconque des revendications précédentes, le traitement comprenant :
la libération de la nicotine en une dose qui diminue dans le temps pour accoutumer graduellement le corps humain à l'absence de nicotine extérieure administrée en fumant du tabac.

8. Produit pour une utilisation selon la revendication 7, le traitement comprenant :
la libération du cannabidiol en une dose qui reste la même pour des produits consécutifs, et préférablement constante pendant la période durant laquelle la nicotine est libérée.

9. Produit pour une utilisation selon la revendication 7 ou 8, le traitement comprenant :
l'utilisation d'une série de patchs sur une période de temps, la dose de nicotine par patch diminuant dans le temps.

10. Produit pour une utilisation selon la revendication 9, la dose de nicotine étant réduite graduellement jusqu'à ce que le patient ne soit plus en état de manque de nicotine et pour le fait de fumer du tabac et arrête d'utiliser le médicament.

11. Produit pour une utilisation selon la revendication 9 ou 10, le traitement comprenant le fait que le patient continue d'utiliser une dose de maintenance de cannabidiol.

12. Produit pour une utilisation selon l'une quelconque des revendications précédentes, comprenant plusieurs cannabinoïdes sans que le produit lui-même ne présente de psychoactivité notable.

13. Kit d'au moins deux produits pour une utilisation selon l'une quelconque des revendications précédentes, la quantité de nicotine ou du sel, de la résine ou du complexe pharmaceutiquement acceptable correspondant(e) et/ou la forme de nicotine ou du sel, de la résine ou du complexe pharmaceutiquement acceptable correspondant(e) étant différente(s) entre les produits pour libérer des doses différentes de nicotine.

14. Kit pour une utilisation selon la revendication 13, comprenant un emballage commun dans lequel les produits sont fournis.
